# EUROPEAN PATENT SPECIFICATION

(11) **EP 1 895 902 B1**
(45) Date of publication and mention of the grant of the patent: **11.11.2009**
(21) Application number: 06701857.2
(22) Date of filing: 17.01.2006
(51) Int. Cl.: A61B 5/053, A61B 5/026

(54) **CEREBRAL PERFUSION MONITOR**
GERÄT ZUR ÜBERWACHUNG DER ZEREBRALEN PERFUSION
APPAREIL DE SURVEILLANCE DE PERFUSION CEREBRALE

(30) Priority: 15.06.2005 WO PCT/IL2005/000632; 15.06.2005 WO PCT/IL2005/000631
(43) Date of publication of application: 12.03.2008
(73) Proprietor: Orsan Medical Technologies Ltd., 46327 Herzelia (IL)
(72) Inventor: REICHMAN, Yosef, 44201 Kfar Saba (IL); POUPKO, Ben, Zion, 74051 Ness Ziona (IL); BEN-ARI, Shlomi, 30550 Binyamina (IL)
(74) Representative: Machtalère, Georges
(86) International application number: PCT/IB2006/050174
(87) International publication number: WO 2006/134501

(56) References cited:
- WO-A-03/059164

## Description

### FIELD OF THE INVENTION

The field of the invention relates to measuring blood flow in the head.

### BACKGROUND OF THE INVENTION

There is a need to measure cerebral blood flow during various medical events and procedures, because any disturbance to the flow of blood to the brain may cause injury to the function of the brain cells, and even death of brain cells if the disturbance is prolonged. Maintaining blood flow to the brain is especially important because brain cells are more vulnerable to a lack of oxygen than other cells, and because brain cells usually cannot regenerate following an injury.

A number of common situations may cause a decrease in the general blood flow to the brain, including arrhythmia, myocardial infarction, and traumatic hemorrhagic shock. A sudden increase in blood flow to the brain may also cause severe damage, and is especially likely to occur in newborn or premature babies, although such an increase may also occur in other patients with certain medical conditions, or during surgery. In all these cases, data regarding the quantity of blood flow in the brain, and the changes in flow rate, may be important in evaluating the risk of injury to the brain tissue and the efficacy of treatment. The availability of such data may enable the timely performance of various medical procedures to increase, decrease, or stabilize the cerebral blood flow, and prevent permanent damage to the brain.

In the absence of a simple means for direct and continuous monitoring of cerebral blood flow, information about changes in cerebral blood flow is inferred indirectly by monitoring clinical parameters which can be easily measured, such as blood pressure. But due to the different relation between blood pressure and cerebral blood flow in different medical conditions, there may be situations in which cerebral blood flow is inadequate even when blood pressure appears to be adequate. Cerebral blood flow may also be inferred indirectly by monitoring neurological function, but since neurological dysfunction is often irreversible by the time it is detected, it is more desirable to detect changes in cerebral blood flow directly, while its effects on brain function are still reversible.

Existing means for measuring cerebral blood flow are complex, expensive, and in some cases invasive, which limits their usefulness. Three methods that are presently used only in research are 1) injecting radioactive xenon into the cervical carotid arteries and observing the radiation it emits as it spreads throughout the brain; 2) positron emission tomography, also based on the injection of radioactive material; and 3) magnetic resonance angiography, performed using a room-sized, expensive, magnetic resonance imaging system, and requiring several minutes to give results. These three methods can only be carried out in a hospital or other center that has the specialized equipment available, and even in a hospital setting it is not practical to monitor patients continuously using these methods.

A forth method, trans-cranial Doppler (TCD) uses ultrasound, is not invasive and gives immediate results. However, TCD fails to give a correct determination of blood flow in about 15% of patients, due to the difficulty of passing sound waves through the cranium, and it requires great skill by professionals who have undergone prolonged training and practice in performing the test and deciphering the results. Another disadvantage of TCD is that it measures only regional blood flow in the brain, and does not measure global blood flow. Doppler ultrasound may also be used to measure blood flow in the carotid arteries, providing an estimate of blood flow to the head, but not specifically to the brain, and not including blood flow to the head through the vertebral arteries. Blood flow through the vertebral arteries is difficult to measure by ultrasound because of their proximity to the vertebrae.

Two additional techniques that are used, generally in research, to measure blood flow in the head and in other parts of the body are electric impedance plethysmography (IPG) and photoplethysmography (PPG), see WO 03/059164 US patent 6,819,950, to Mills describes the use of PPG to detect carotid stenosis, among other conditions. US patent 5,694,939, to Cowings describes biofeedback techniques for controlling blood pressure, which include the use ofIPG in the lower leg and PPG in the finger. US patent 5,396,893, to Oberg et al. states that PPG is superior to IPG for monitoring patients' cardiac and respiration rates. US patent 6,832,113, to Belalcazar describes the use of either IPG or PPG to measure blood flow, for purposes of optimizing a cardiac pacemaker. US patent 6,169,914, to Hovland et al. describes the use of various types of sensors, including IPG and PPG, for monitoring female sexual arousal with a vaginal probe, and describes using different types of sensors in combination.

US patent 6,413,223, to Yang et al. describes a probe, used on the finger, which contains two PPG sensors and one IPG sensor. The combined data from the three sensors, analyzed using a mathematical model of arterial blood flow, provides a more accurate measurement of blood flow than would be obtained by using IPG or PPG alone.

J. H. Seipel and J. E. Floam, in J. Clinical Pharmacology 15, 144-154 (1975) present the results of a clinical study of the effects of a drug, betahistidine, on cerebral, cranial, scalp and calf blood circulation. Rheoencephalography (REG), a form of IPG, was used to measure the amplitude of cerebral blood flow.

### SUMMARY OF THE INVENTION

An aspect of some embodiments of the invention relates to determining cerebral blood flow from IPG data, using the data only from selected cardiac cycles, and discarding the data from other cardiac cycles, according to characteristics of the IPG data and/or other data, for example EKG data. Optionally, the IPG data is obtained from electrodes placed on the head or in ears, for example as described in any of the above mentioned related patent applications. The cerebral blood flow is determined from a combination of IPG data and PPG data, and characteristics of the IPG data, the PPG data, other data, or any combination of them, are used to select cardiac cycles from which the IPG and PPG data is used. Optionally, the PPG data is obtained from PPG sensors placed on the head or in the ears, for example as described in any of the above mentioned related patent applications. Optionally, these characteristics comprise the duration of the cardiac cycle, and data is used for cardiac cycles that have similar duration, while cardiac cycles with very different durations are discarded. Additionally or alternatively, the characteristics comprise a cross-correlation between the signal for each cardiac cycle and the following (or preceding) cardiac cycle, for the IPG signal and/or for the PPG signal. For example, data is used for a cardiac cycle only if the cross-correlation exceeds a threshold, for the IPG signal or for the PPG signal, or only if the cross-correlation exceeds a threshold for both the IPG and PPG signals.

As aspect of some embodiments of the invention relates to reducing breathing artifacts from IPG data and/or PPG data, before using the IPG data, or a combination of the IPG and PPG data, to measure cerebral blood flow. Breathing artifacts are reduced, for example, by adjusting the data differently in each cardiac cycle, such that the data at a particular phase in the cardiac cycle, or an average of the data over a particular range of phases of the cardiac cycle, always has a fixed value. Optionally, breathing artifacts are substantially removed from the IPG data and/or from the PPG data, for example the cerebral blood flow calculated from the IPG and PPG data varies by less than 10% as a function of phase of the breathing cycle, on average over many breathing cycles. Optionally, the particular phase in the cardiac cycle is the diastolic phase, as indicated, for example, by the peak of the R-wave, or as indicated by a minimum in the IPG signal or the PPG signal.

An aspect of some embodiments of the invention relates to using a ratio of a slope, optionally a maximum slope of the IPG signal to a slope, optionally a maximum slope of the PPG signal, as a measure of cerebral blood flow. This slope is believed to be strongly correlated with the blood inflow. Optionally, the maximum slope used for both the IPG and PPG signals is the maximum slope of the leading edge, following the diastolic phase. Alternatively, the maximum slope used for one or both signals is the slope of maximum absolute value at the trailing edge, preceding the diastolic phase. Optionally, the maximum slope is normalized, for example by dividing it by a measure of the amplitude of the signal for that cardiac cycle. Optionally, the resulting measurement of cerebral blood flow is then smoothed by using an average over time. For example, a running average over time is used, with a fixed time interval, for example a few seconds, or with a fixed number of cardiac cycles. Optionally, the smoothing is done over a time interval that varies with time, adapting to characteristics of the signal.

There is thus provided, in accordance with an exemplary embodiment of the invention, a method of estimating cerebral blood flow by analyzing IPG and PPG signals of the head, the method comprising:
a) finding a maximum slope or most negative slope or the IPG signal, within at least a portion of the cardiac cycle;
b) finding a maximum slope or most negative slope of the PPG signal, within at least a portion of the cardiac cycle;
c) finding a ratio of the maximum or most negative slope of the IPG signal to the maximum or most negative slope of the PPG signal; and
d) calculating a cerebral blood flow indicator from the ratio.

Optionally, finding the maximum or most negative slope comprises finding the maximum slope, for both the IPG and PPG signals, and finding a ratio comprises finding a ratio of the maximum slopes.

Optionally, the maximum slopes are maximums within a leading portion of the cardiac cycle.

Alternatively, finding the maximum or most negative slope comprises finding the most negative slope, for both the IPG and PPG signals, and finding a ratio comprises finding a ratio of the most negative slopes.

Optionally, the most negative slopes are most negative within a trailing portion of the cardiac cycle.

In an embodiment of the invention, the maximum or most negative slope of at least one of the signals is normalized to a measure of the amplitude of said signal.

Optionally, the measure of the amplitude is the peak-to-peak amplitude of said signal over the cardiac cycle.

Alternatively, the measure of the amplitude is an average value of said signal over the cardiac cycle.

Optionally, the PPG signal comes from a PPG sensor on the left side of the head. Additionally or alternatively, the PPG signal comes from a PPG sensor on the right side of the head.

Additionally or alternatively, the PPG signal is an average of signals from a PPG sensor on the left side of the head and a PPG sensor on the right side of the head.

There is further provided, in accordance with an exemplary embodiment of the invention, a method of estimating time-varying cerebral blood flow, comprising:
a) obtaining a time-varying IPG signal of the head;
b) obtaining a time-varying PPG signal of the head;
c) using the IPG and PPG signals to calculate a time-varying indicator for cerebral blood flow; and
d) performing data processing on one or more of the IPG signal, the PPG signal, and the cerebral blood flow indicator, to reduce noise or artifacts or both.

In an embodiment of the invention, performing data processing comprises discarding data of the IPG signal, the PPG signal, or both, for cardiac cycles which meet one or more criteria for discarding.

Optionally, the criteria comprise having a duration outside an expected range.

Optionally, the expected range has a maximum between 1.3 and 2 times an average duration of cardiac cycles.

Additionally or alternatively, the criteria comprise one or both of the IPG signal and the PPG signal having a cross-correlation below a threshold, between that cardiac cycle and the following cardiac cycle.

Additionally or alternatively, the criteria comprise one or both of the IPG signal and the PPG signal having a cross-correlation below a threshold, between that cardiac cycle and the preceding cardiac cycle.

Optionally, the threshold is between +0.5 and +0.8.

In an embodiment of the invention, performing data processing comprises reducing breathing artifacts in the IPG signal, the PPG signal, or both.

Optionally, calculating the cerebral blood flow indicator comprises using the method according to an exemplary embodiment of the invention.

In an embodiment of the invention, performing data processing comprises smoothing the cerebral blood flow indicator.

Optionally, smoothing comprises finding an average over a time interval.

Optionally, smoothing comprises using a time scale that is adjusted adaptively, depending on behavior of the cerebral blood flow indicator as a function of time.

### BRIEF DESCRIPTION OF THE DRAWINGS

Exemplary embodiments of the invention are described in the following sections with reference to the drawings. The drawings are not necessarily to scale and the same reference numbers are generally used for the same or related features that are shown on different drawings.
Fig. 1 is a flowchart of a method for finding cerebral blood flow, according to an exemplary embodiment of the invention;
Figs. 2A-2D schematically show graphs of IPG and PPG signals, with breathing artifacts and with the breathing artifacts removed, according to an exemplary embodiment of the invention;
Fig. 3 schematically shows a graph of IPG and PPG signals, during good and bad cardiac cycles, according to an exemplary embodiment of the invention;
Fig. 4 schematically shows a graph of a calculated cerebral blood flow indicator as a function time during an endarterectomy procedure, according to an exemplary embodiment of the invention;
Fig. 5 schematically shows a graph of the cerebral blood flow indicator shown in Fig. 4, showing the effect of including all of the cardiac cycles, and including only the good cardiac cycles, according to an exemplary embodiment of the invention;
Fig. 6 schematically shows a graph of the cerebral blood flow indicator shown in Fig. 4, which was smoothed over time, together with the values of the indicator before smoothing, according to an exemplary embodiment of the invention;
Fig. 7 schematically shows a graph of a calculated cerebral blood flow indicator of a subject as a function of time, according to an exemplary embodiment of the invention, during a test in which the cerebral blood flow was increased by having the subject breath air with an increased level of carbon dioxide; and
Fig. 8 schematically shows a graph of a calculated cerebral blood flow indicator for the left and right hemispheres of a subject's brain as a function of time, according to an exemplary embodiment of the invention, during a test in which the subject performed a cognitive task which increased the left hemisphere blood flow.

### DETAILED DESCRIPTION OF EXEMPLARY EMBODIMENTS

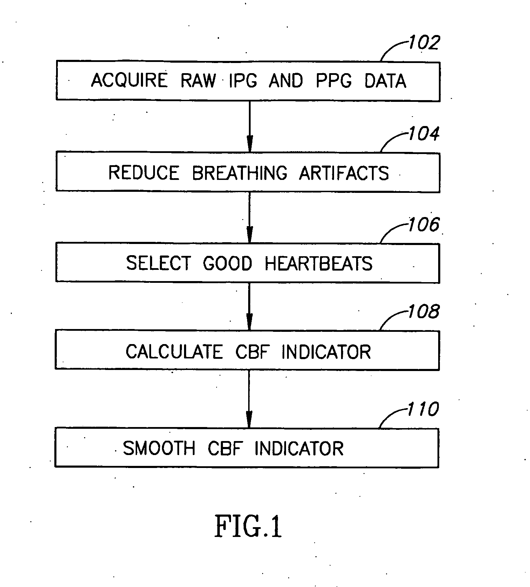

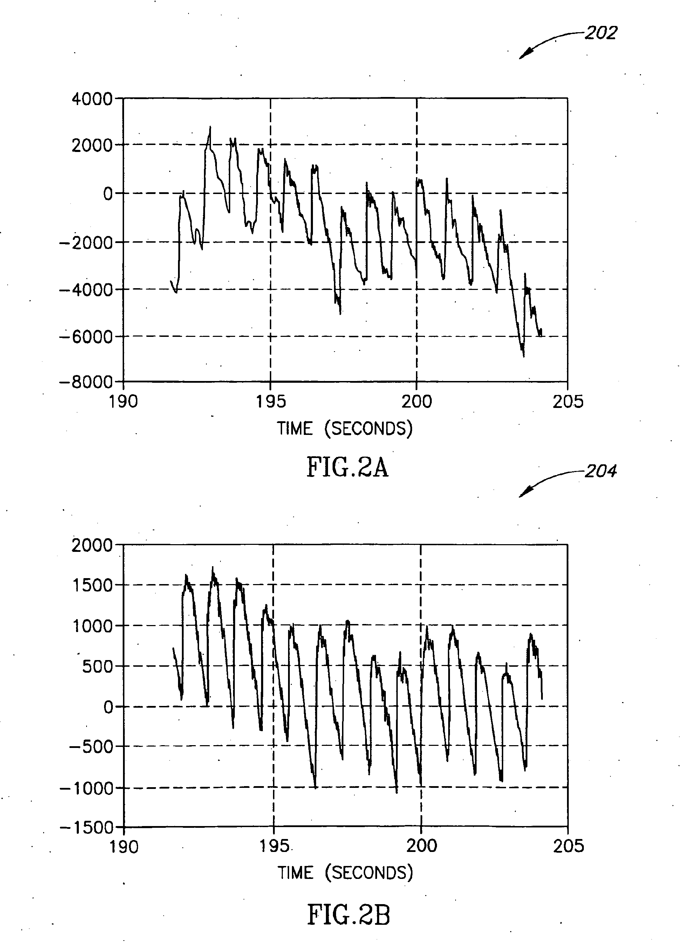

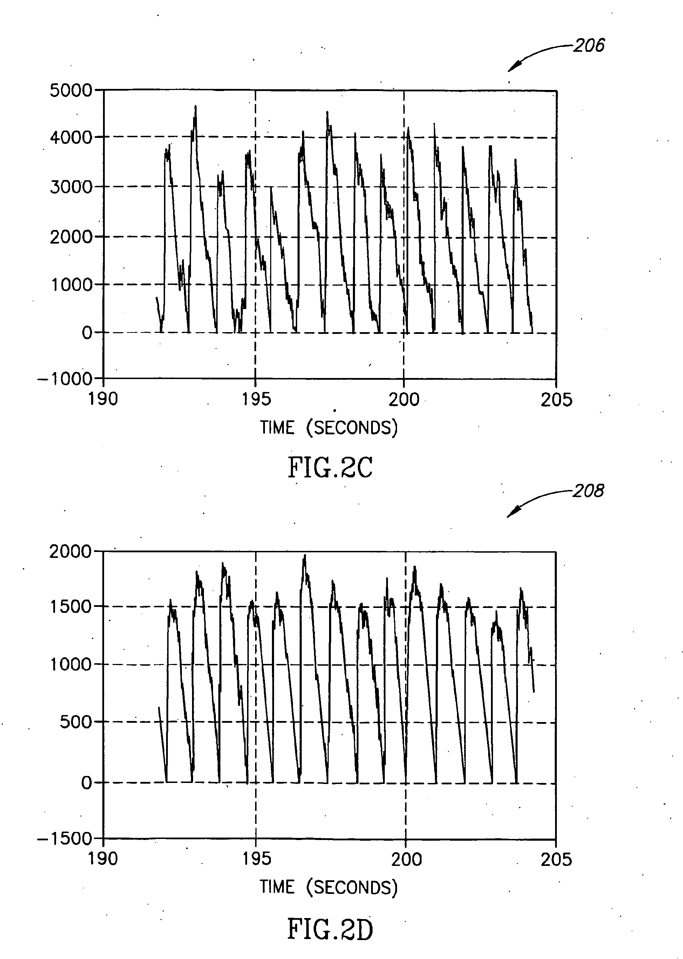

Fig. 1 shows a flowchart 100, outlining a method for finding cerebral blood flow (CBF) according to an exemplary embodiment of the invention. The different steps in flowchart 100 will be described with reference to graphs of data, shown in Figs. 2, 3, and 4.

At 102, raw IPG and PPG data of the head is acquired. The data is acquired, for example, using any of the methods described in any of the above mentioned related patent applications or the patents, and publications as referenced in the Background, or any other methods known in the art for acquiring IPG and PPG data of the head. For example, combined sensors, incorporating both electrodes for IPG and optical sensors for PPG, are used, or separate sensors are used. Optionally, the IPG electrodes are designed to be of a size and shape, and are positioned on the head, so as to obtain IPG data that is relatively more sensitive to the impedance of the interior of the skull, and relatively less sensitive to the impedance of the scalp, as described in the above mentioned patent applications. Examples of raw IPG and PPG signals as a function of time are shown respectively in plots 202 and 204 of Figs. 2A and 2B.

Optionally, there is more than one PPG sensor, for example, there are two PPG sensors, one on each side of the head. Optionally, the two PPG sensors are located respectively on the left and right sides of the forehead. In any of the methods described herein for using the PPG signal, either the PPG signal from the left side of the head or the PPG signal from the right side of the head may be used, or an average of the two PPG signals may be used, possibly a weighted average.

At 104, the raw IPG and/or PPG signals are optionally conditioned to reduce breathing artifacts. This is done, for example, by adjusting the signals so that the minimum value for each cardiac cycle has a constant value, set at zero in Figs. 2C and 2D. Methods of defining what constitutes a single cardiac cycle are described below, in connection with 106. The resulting conditioned IPG signal, shown in plot 206, and conditioned PPG signal, shown in plot 208, are nearly free of breathing artifacts. There is no apparent correlation of the IPG signals and PPG signals with the breathing cycle visible to a casual viewer of plots 206 and 208, and any remaining correlation of the IPG signal and/or the PPG signal to the breathing cycle optionally results in less than a 10% effect on the calculated cerebral blood flow. Optionally, the values between minima are reduced by the average value or an interpolated value of the adjacent minima.

Optionally, at 106, "good" cardiac cycles are selected, and data from other cardiac cycles is discarded. Optionally, one or more of three criteria are used for discarding data from some cardiac cycles. The first criterion concerns how much the duration of the cardiac cycle differs from an average duration. The second and third criteria concern how much the form of the signal for a given cardiac cycle differs from the form of the signal for the following (or preceding) cardiac cycle, for the IPG signal and the PPG signal respectively. Data from cardiac cycles which satisfy one or more of these criteria is likely to have a high noise level which distorts the signals, or may correspond to an irregular heartbeat which does not provide a typical value for cerebral blood flow. The inventors have found that discarding data for cardiac cycles which satisfy any one of these three criteria is particularly useful for determining an accurate measure of cerebral blood flow. Optionally, data is discarded only if the cardiac cycle satisfies two of these criteria, or all three criteria. Optionally, only one of these three criteria is used as a criterion to discard data. Optionally, only two of the criteria are used, and the data is discarded if either of the two criteria is satisfied. Optionally, all three criteria are used, and the data is discarded if any of the criteria are satisfied. Other criteria for determining if the data for a particular cardiac cycle should be discarded will occur to a person of skill in the art.

The duration of the cardiac cycle is determined, for example, using EKG data, and defined as the time from the peak of one R-wave to the peak of the next R-wave. To be considered as the peak of an R-wave, the peak optionally must meet certain criteria. For example, the peak falls between 0.3 seconds and 1.5 seconds of the peak of the previous R-wave. If there is more than one local peak within this time interval, the peak of the R-wave is optionally found by finding the peak which most resembles the expected amplitude and time interval for the peak of an R-wave. The expected amplitude and time interval are based, for example, on the amplitude and time interval for the previous peak of an R-wave, or on a running average of past values. Optionally, instead of or in addition to using the peak of the R-wave to define the duration of the cardiac cycles, IPG data and/or PPG data is used. For example, the duration of a cardiac cycle is defined as the time from one local minimum (or maximum) to the next local minimum (or maximum) in the IPG and/or the PPG signal, or as the time from one local maximum (or minimum) in slope of the signal to the next local maximum (or minimum) in slope. Optionally, the local minimum or maximum in the IPG or PPG signal, or in the slope of the IPG or PPG signal, must meet certain criteria, for example criteria similar or identical to the criteria described above for using the peak of the R-wave. Optionally, data is discarded for those cardiac cycles that have a duration outside an expected range. Optionally, the maximum of the expected range is between 1.3 and 2 times an average duration of a cardiac cycle. For example, the maximum is 1.65 times the average duration. Alternatively, the maximum of the expected range is less than 1.3 times the average duration. Optionally, the minimum of the expected range is less than 0.7 times the average duration. Alternatively, the minimum of the expected range is more than 0.7 times the average duration. Optionally, there is no explicit minimum to the range, although there may be a minimum duration for any cardiac cycle, due to the way that cardiac cycles are defined, as described above.

The "average duration of a cardiac cycle" described above is optionally the median or the mode of the durations of the cardiac cycles. A potential advantage of using the median or the mode, rather than the mean, is that the median and the mode are relatively insensitive to the values of outliers that may represent noise in the data rather than real durations of cardiac cycles. Alternatively, the "average duration of a cardiac cycle" is the mean of the durations of cardiac cycles. Optionally, the "average duration of a cardiac cycle" is a running average, for example over several cardiac cycles, or over several tens of cardiac cycles. Using a running average for the average duration of a cardiac cycle has the potential advantage of adjusting the average duration to real changes in the patient's pulse rate, due to physiological changes over time. Optionally, a fixed value is used in place of the "average duration of a cardiac cycle," optionally adjusted to the patient, or the fixed value is based on a pulse rate determined for that patient.

How much the form of the signal (either the IPG or PPG signal) for a given cardiac cycle differs from the signal for the following cardiac cycle, is determined, for example, by the cross-correlation between the signals for the two cardiac cycles. Optionally, if the cross-correlation is less than some threshold, for one or both of the IPG and PPG signals, then the data for that cardiac cycle is discarded. Optionally, the threshold is between +0.5 and +0.8, for example the threshold is +0.7. Optionally, instead of using the cross-correlation between a cardiac cycle and the following cardiac cycle for the criterion, the criterion is based on the cross-correlation between the cardiac cycle and the previous cardiac cycle. Alternatively, the data is discarded only if either of these two cross-correlations is less than the threshold, or only if both cross-correlations are less than the threshold. Optionally, the data is discarded only if the cross-correlation (whichever one is used) is below the threshold for both the IPG and PPG signals. Alternatively, the data is discarded only if the cross-correlation is below the threshold for the IPG signal, or only if the cross-correlation is below the threshold for the PPG signal.

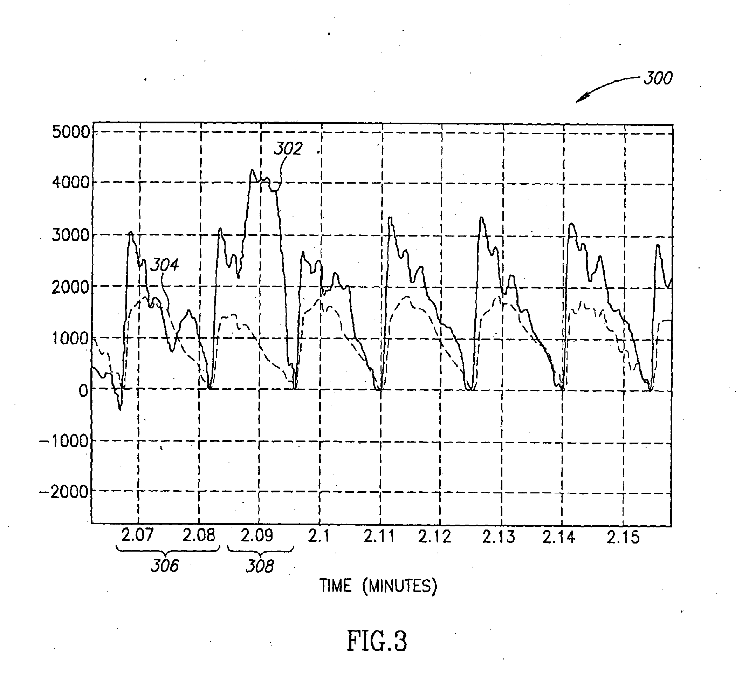

Fig. 3 shows a plot 300 of conditioned IPG data 302 (solid curve) and conditioned PPG data 304 (dashed curve). For the first two cardiac cycles, in time intervals 306 and 308, the cross-correlation between that cardiac cycle and the next is relatively low for the IPG data, apparently because of noise in the IPG data, and the data for these cardiac cycles is discarded. For the remaining cardiac cycles, the cross-correlation between that cardiac cycle and the next is relatively high, for both the IPG and the PPG signals, and the data for these cardiac cycles is not discarded.

At 108, a CBF indicator is calculated from the IPG and/or PPG data for the cardiac cycles for which the data has been kept. In an exemplary embodiment of the invention, the CBF indicator is found, for each such cardiac cycle, by taking the ratio of the maximum slope of the IPG signal to the maximum slope of the PPG signal. Optionally, the maximum slopes are not necessarily maximums over the whole cardiac cycle, but arc maximums over a leading edge portion of the cardiac cycle, following the diastolic phase. It should be understood that the magnitudes of both the IPG and PPG signals, and hence the maximum slopes of both signals, in general may be sensitive to various factors. These factors include the exact position of the electrodes and PPG sensors on the patient's head, how good the contact is with the skin, and the thickness of the patient's skin and of the fatty layer beneath the patient's skin at the location of the electrodes and elsewhere on the patient's head. The ratio of the maximum slopes of the IPG and PPG signals may not provide an absolute measure of cerebral blood flow, but may provide only a relative measure of cerebral blood flow. Optionally, the measure of cerebral blood flow is calibrated by observing its value at a time when the patient is known to have adequate cerebral blood flow, for example before surgery, at a time when the patient is conscious and his mental state can be assessed by asking him questions. Optionally, the electrodes and PPG sensors are not removed or repositioned once the measure of cerebral blood flow has been calibrated, until surgery has been completed, for example.

Because the arteries in the brain are generally greater in diameter than the arteries of the skin of the face and the scalp, the blood volume in the brain generally increases sooner and faster at the beginning of the systolic phase, than the blood volume in the skin does. Because the IPG signal is sensitive both to the blood volume in the brain and the blood volume in the skin, while the PPG signal is sensitive only to the blood volume in the skin, the IPG signal generally rises sooner and faster, at the beginning of the systolic phase, than the PPG signal. The maximum slope of each signal is a measure of how fast this rise occurs, and how high the rise goes. The maximum slope of the IPG signal is a measure of a weighted sum of the blood flow in the brain and the blood flow in the skin, while the maximum slope of the PPG signal is a measure of the blood flow in the skin alone. The maximum slopes of the IPG and PPG signals may be better measures of blood flow in these regions than the peak-to-peak amplitude of the IPG and PPG signals, which measure changes in blood volume. The change in blood volume depends on the difference between blood flow into and out of a region. However, the peak-to-peak amplitudes of the IPG and PPG signals may also be useful for measuring cerebral blood flow.

Another useful measure of blood flow, for either the IPG signal or the PPG signal or both, is the maximum slope of the signal normalized to a measure of the amplitude of the signal. For example, the maximum slope is normalized by dividing by the peak-to-peak amplitude of the signal for that cardiac cycle. Alternatively, the maximum slope is normalized by dividing by a difference between an average value of the signal, possibly a weighted average value, and the minimum value of the signal, for that cardiac cycle. Optionally the weighted average value includes both positive and negative weights, for example the weighted average value is a Fourier component of the signal at the cardiac cycle frequency. Optionally, the normalization is to the area of the signal for example between successive minima.

The inventors have found that the ratio of maximum slope of the IPG signal to maximum slope of the PPG signal (either normalizing the maximum slopes or not) is often well correlated with blood flow rate in the brain in certain circumstances, as determined independently by other means, for example TCD. For example, in certain circumstances, the brain increases cerebral blood flow by constricting peripheral arteries which affect blood flow to the scalp and to the skin of the face. In these cases, an increase in cerebral blood flow correlates with a decrease in blood flow in the skin, and the ratio of maximum slope of IPG signal to maximum slope of PPG signal may be well correlated with cerebral blood flow.

In circumstances where cerebral blood flow is reduced by blocking or bleeding of an artery on one side of the head, the peripheral blood flow on the other side of the head may remain relatively constant. In these cases, particularly if the PPG signal is measured on the other side of the head from the affected artery, the ratio of the maximum slope of the IPG signal to the maximum slope of the PPG signal may also be well correlated with cerebral blood flow. Even if the PPG signal is taken on the same side of the head as the affected artery, the ratio of maximum slopes may be reasonably well correlated with cerebral blood flow, perhaps because collateral arteries may redistribute blood from one side of the head to the other.

In other circumstances, different measures of cerebral blood flow may be more useful. For example, if total blood flow to the head is reduced because of a decrease in blood pressure, then the brain may compensate by constricting peripheral arteries, reducing blood flow in the skin more than in the brain. In this case, the maximum slope of the IPG signal alone, or a weighted difference in maximum slopes between the IPG and PPG signals, may be a better measure of cerebral blood flow than the ratio of the maximum slopes.

In some embodiments of the invention, a different formula is used for finding the CBF indicator. For example, instead of using ratios of the maximum slopes of the IPG and PPG signals, the ratio of the minimum (most negative) slopes is used instead, with the slopes either normalized to a measure of the amplitudes or not. Optionally, the most negative slopes are not necessarily the most negative over the whole cardiac cycle, but only over a trailing edge portion of the cardiac cycle, following the systolic phase. The fall in blood volume after the systolic phase, like the rise in blood volume after the diastolic phase, may be faster for the brain than for the skin. The ratio of minimum slopes of the IPG and PPG signals may be related to cerebral blood flow in a similar way to the ratio of maximum slopes. Alternatively, when taking the ratio of slopes; the maximum slope is used for one of the signals and the minimum slope (or its absolute value) is used for the other signal.

Alternatively or additionally, the CBF indicator is found by subtracting a weighted PPG signal from the IPG signal, and then taking the maximum slope of the difference signal. Optionally, the weighting factor is determined by requiring a slope of the trailing edge of the weighted PPG signal, for example an average slope of the trailing edge, or a steepest slope of the trailing edge, to be equal to the corresponding slope of the IPG signal. This choice of weighting factor may be appropriate if the trailing edge of the IPG signal is dominated by blood flow in the skin. The resulting CBF indicator has the potential advantage that it may better indicate changes in cerebral blood flow caused by a decrease in blood pressure, which decreases blood flow in both the brain and the skin. On the other hand, a CBF indicator based on the ratios of the slopes of two signals may be less sensitive to noise in the signals than a CBF indicator based on the slope of a difference between two signals.

Optionally, the CBF indicator is based only on the IPG signal, or only on the PPG signal. For example, the CBF indicator is the peak-to-peak amplitude of one of the signals in each cardiac cycle, or the maximum or minimum slope of one of the signals, or the maximum or minimum slope normalized to an amplitude of the signal, in each cardiac cycle.

In 110, the CBF indicator signal is averaged over time, using any known algorithm for temporal smoothing. Optionally, the averaging is done over a time scale of several seconds, for example over 5, 10, or 20 seconds, or over a plurality of cardiac cycles, for example over 5, 10, or 20 cardiac cycles. Optionally, the time scale for the smoothing varies adaptively, depending on the data being smoothed. For example, the smoothing comprises averaging the data over a time interval which is adjusted upward if a linear extrapolation makes a good prediction about where the next data point will be, and is adjusted downward if a linear extrapolation makes a poor prediction about where the next data point will be.

Optionally, instead of, in addition to, averaging the CBF indicator over a plurality of cardiac cycles, the IPG signals for each of a plurality of cardiac cycles are superimposed and averaged together, and the same is optionally done for the PPG signal, before finding the CBF indicator in 108, using any of the methods described above.

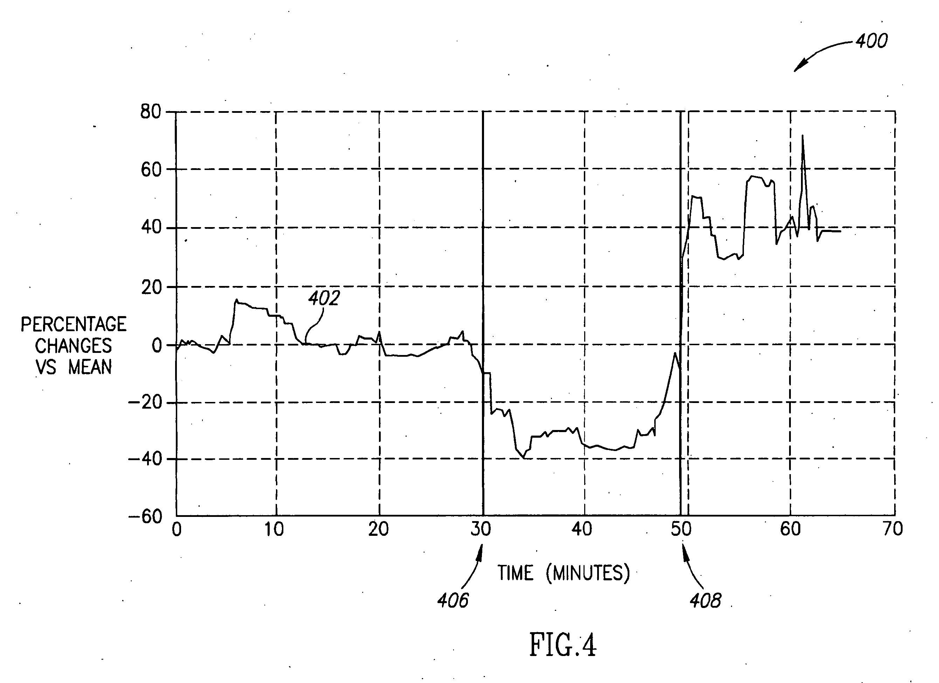

Fig. 4 shows a graph 400, with a plot of a smoothed CBF indicator signal 402 as a function of time. The CBF indicator was calculated by taking the ratio of the normalized maximum slope of the IPG signal to the normalized maximum slope of the PPG signal, with the normalization done using the peak-to-peak amplitude of each signal. The smoothing of the CBF indicator was done by averaging over an adaptively varying time interval, as described above. The IPG and PPG signals were measured on a patient undergoing an endarterectomy, in which the common, internal, and external carotid arteries on one side of the neck were clamped between time 406 and time 408, while the arteries were cleared of plaque. The PPG data used was taken from the side of the head opposite to the clamped arteries. The CBF indicator signal 402 decreases at time 406, primarily due to a decrease in the IPG signal, when the arteries are clamped and blood flow to that side of the head, and to the brain as a whole, is reduced. At time 408, when the clamped arteries are released, the CBF indicator signal 402 increases, primarily due to an increase in the IPG signal. The CBF indicator signal is higher after time 408 than it was before the arteries were clamped, because the arteries cleared of plaque allow greater cerebral blood flow than before.

It should be noted that this method of calculating the CBF indicator has been found by the inventors to generally give the best results for cerebral blood flow during an endarterectomy, of the methods that have been tested. However some other methods of calculating the CBF indicator, including using the PPG signal from the same side of the head as the clamped arteries, have also been found to give a fairly good indication of cerebral blood flow during endarterectomy.

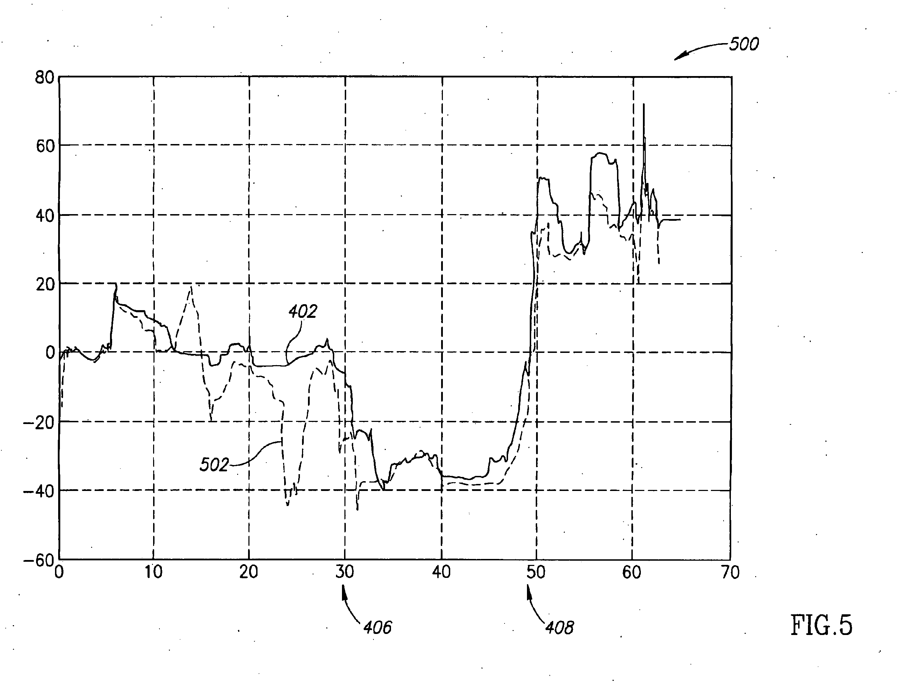

Fig. 5 shows a graph 500, illustrating the effect on the CBF indicator of discarding bad cardiac cycles. The CBF indicator signals shown in graph 500 were calculated from the same data used in Fig. 4. CBF indicator signal 402, shown as a solid line, was calculated using only "good" cardiac cycles, and is the same as signal 402 shown in Fig. 4. Good cardiac cycles were defined as those for which the duration of the cardiac cycle was less than 1.65 times the median duration of all cardiac cycles, and for which both the IPG and PPG signals had a cross-correlation of at least +0.7 between that cardiac cycle and the following cardiac cycle. CBF indicator signal 502, shown as a dashed line, was calculated in the same way, but including signal data from all cardiac cycles. Although signal 502 shows the same general trend as signal 402, decreasing while the arteries are clamped and returning to an even higher level after the arteries are released, signal 502 shows considerably more noise than signal 402.

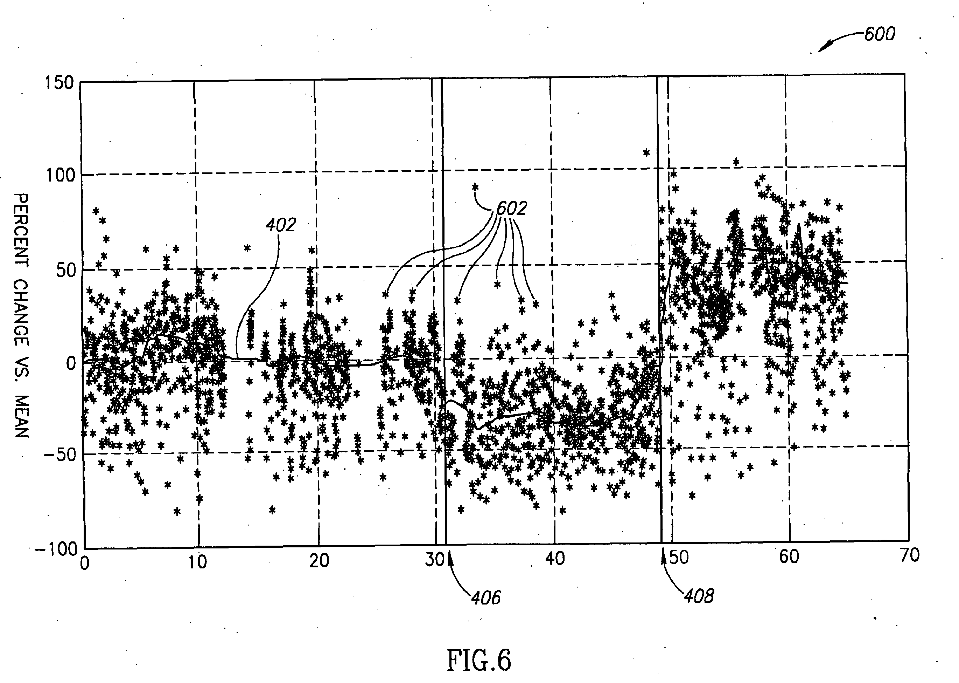

Fig. 6 shows a graph 600, illustrating the effect of smoothing on the CBF indicator. Smoothed CBF indicator 402 plotted in graph 600 is the same as signal 402 plotted in Figs. 4 and 5. A large number of small stars 602 show the values of the CBF indicator for individual cardiac cycles, which show a much higher level of noise than smoothed signal 402.

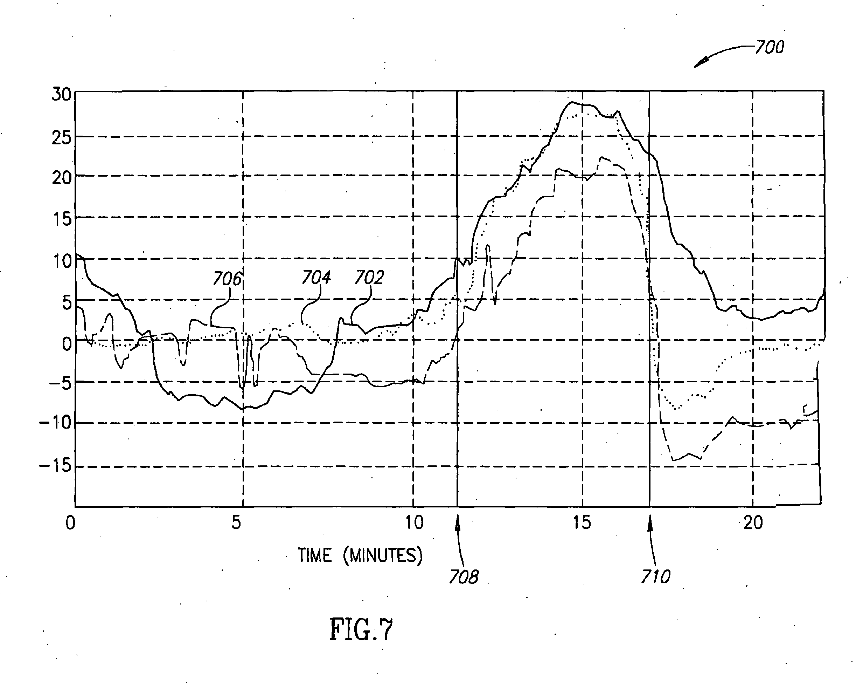

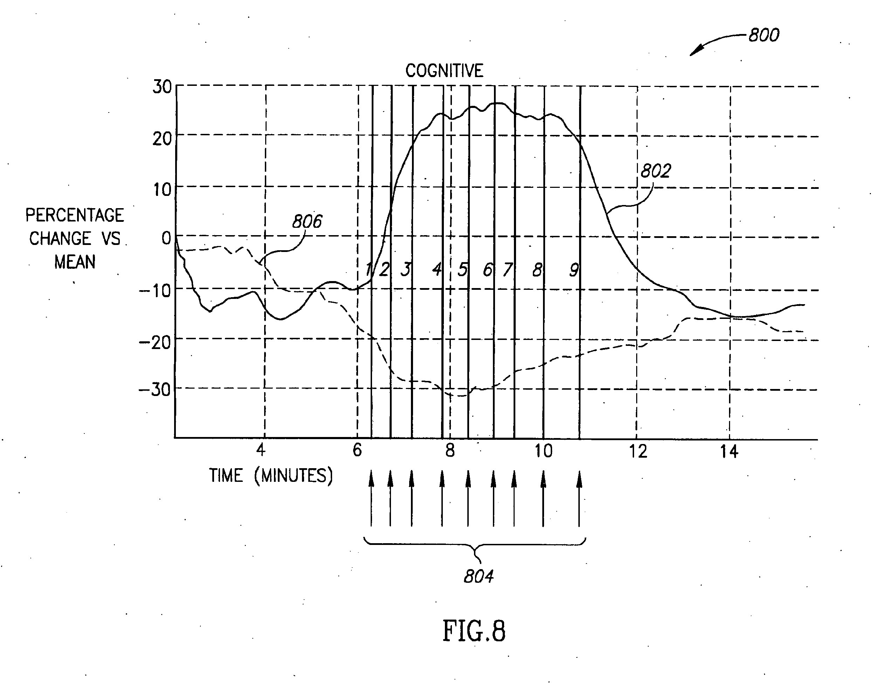

Figs. 7 and 8 show the results of two other tests that were performed by the inventors to verify the usefulness of the CBF indicator signal, using healthy volunteers. In these tests, CBF indicators 702 (in Fig. 7) and 802 and 806 (in Fig. 8) were defined as the ratio of the maximum slope of the IPG signal to the maximum slope of one of the PPG signals, but the maximum slopes were not normalized to the amplitudes of the respective signals. This method of calculating the CBF indicator generally gave better results in these two tests, than using normalized maximum slopes did. The smoothing method, and the definition of "good" cardiac cycles, were the same as for CBF indicator 402 in Figs. 4-6.

In the test used to generate the data plotted in graph 700 in Fig. 7, the subject breathed normal air until time 708. Between time 708 and time 710, the subject breathed from a closed bag, resulting in an increased level of carbon dioxide, a procedure which is known to provoke an increase in cerebral blood flow. After time 710, the subject returned to breathing normal air. The measured level of carbon dioxide in the gas that the subject exhaled, relative to a typical normal exhaled carbon dioxide partial pressure of 40 mm Hg, is plotted in graph 700 as signal 704. As expected, CBF indicator 702 rises when the level of carbon dioxide rises, and falls again when the level of carbon dioxide falls. The change in CBF indicator 702 is due largely to changes in the PPG signal, which decreases when the level of carbon dioxide increases, because the brain constricts peripheral arteries of the head, in order to assure a continued adequate supply of oxygen to the brain. A smoothed TCD signal 704, a standard indicator for cerebral blood flow, shows a similar rise when the level of carbon dioxide rises.

Fig. 8 illustrates the effect of cognitive activity on cerebral blood flow. CBF indicator 802 was calculated using the PPG signal from the left side of the head. CBF indicator 802 should indicate specifically the blood flow in the left side of the brain, since the brain is known to constrict or relax the peripheral arteries separately on either the left or the right side of the head, in order to regulate the blood flow on the corresponding sides of the brain. The subject was presented with nine multiplication problems, and asked to solve them in his head, at the times indicated by arrows 804. Mental arithmetic is known to be an activity primarily of the left side of the brain, and during the time the subject was solving the problems, CBF indicator 802 showed an increase in left cerebral blood flow, with about a two minute delay. By contrast, CBF indicator 806, which was calculated using the PPG signal from the right side of the head, shows no such increase, indicating that there was no increase in right cerebral blood flow during this period. CBF indicator 806 may even show a slight decrease during this period. The changes in both CBF indicators are due primarily to changes in the PPG signal.

## Claims

1. A method of estimating cerebral blood flow by analyzing IPG i.e., impedance plethysmography, and PPG i.e., photoplethysmograply signals of the head, the method comprising:
a) finding a maximum slope or most negative slope of the IPG signal, within at least a portion of the cardiac cycle;
b) finding a maximum slope or most negative slope of the PPG signal, within at least a portion of the cardiac cycle;
c) finding a ratio of the maximum or most negative slope of the IPG signal to the maximum of most negative slope of the PPG signal; and
d) calculating a cerebral blood flow indicator from the ratio.

2. A method according to claim 1 wherein finding the maximum or most negative slope comprises finding the maximum slope, for both the IPG and PPG signals, and finding a ratio comprises finding a ratio of the maximum slopes.

3. A method according to claim 2, wherein the maximum slopes are maximums within a leading portion of the cardiac cycle.

4. A method according to claim 1 wherein finding the maximum or most negative slope comprises finding the most negative slope, for both the IPG and PPG signals, and finding a ratio comprises finding a ratio of the most negative slopes.

5. A method according to claims 4, wherein the most negative slopes are moat negative within a trailing portion of the cardiac cycle.

6. A method according to any of the preceding claims, wherein the maximum or most negative slope of at least one of the signals is normalized to a measure of the amplitude of said signal.

7. A method according to claim 6, wherein the measure of the amplitude is the peak-to-peak amplitude of said signal over the cardiac cycle.

8. A method according to claim 6, wherein the measure of the amplitude is an average value of said signal over the cardiac cycle.

9. A method according to any of the preceding claims, wherein the PPG signal comes from a PPG sensor on the left side of the head.

10. A method according to any of claims 1-8, wherein the PPG signal comes from a PPG sensor on the right side of the head.

11. A method according to any of claims 1-8, wherein the PPG signal is an average of signals from a PPG sensor on the left side of the head and a PPG sensor on the right side of the head.

12. A method of estimating time-varying cerebral blood flow, comprising:
a) obtaining a time-varying IPG, i.e., impedance plethysmography, signal of the head;
b) obtaining a time-varying PPG i.e., photoplethysmography, signal of the head;
c) using the IPG and PPG signals to calculate a time-varying indicator for cerebral blood flow; and
d) performing data processing on one or more of the IPG signal, the PPG signal, and the cerebral blood flow indicator, to reduce noise or artifacts or both.

13. A method according to claim 12, wherein performing data processing comprises discarding data of the IPG signal, the PPG signal, or both, for cardiac cycles which meet one or more criteria for discarding.

14. A method according to claim 13, wherein the criteria comprise having a duration outside an expected range.

15. A method according to claim 14, wherein the expected range has a maximum between 1.3 and 2 times an average duration of cardiac cycles.

16. A method according to any of claims 13-15, wherein the criteria comprise one or both of the IPG signal and the PPG signal having a cross-correlation below a threshold, between that cardiac cycle and the following cardiac cycle.

17. A method according to any of claims 13-15, wherein the criteria comprise one or both of the IPG signal and the PPG signal having a cross-correlation below a threshold, between that cardiac cycle and the preceding cardiac cycle.

18. A method according to claim 16 or claim 17, wherein the threshold is between +0.5 and +0.8.

19. A method according to any of claims 12-18, wherein performing data processing comprises reducing breathing artifacts in the IPG signal, the PPG signal, or both.

20. A method according to any of claims 12-19, wherein calculating the cerebral blood flow indicator comprises using the method of any of claims 1-11.

21. A method according to any of claims 12-20, wherein performing data processing comprises smoothing the cerebral blood flow indicator.

22. A method according to claim 21, wherein smoothing comprises finding an average over a time interval.

23. A method according to claim 21 or claim 22, wherein smoothing comprises using a time scale that is adjusted adaptively, depending on behavior of the cerebral blood flow indicator as a function of time.

## Patentansprüche

1. Verfahren zum Schätzen eines zerebralen Blutflusses durch die Analyse von IPG-, d.h. Impedanzplethysmographie, und PPG-, d.h. Photoplethysmographie, Signalen des Kopfes, wobei das Verfahren folgende Schritte umfasst:
a) Finden einer maximalen Steigung oder einer größten negativen Steigung des IPG-Signals, in mindestens einem Abschnitt des Herzzyklus;
b) Finden einer maximalen Steigung oder einer größten negativen Steigung des PPG-Signals, in mindestens einem Abschnitt des Herzzyklus;
c) Finden eines Verhältnis zwischen der maximalen oder größten negativen Steigung des IPG-Signals und der maximalen oder größten negativen Steigung des PPG-Signals; und
d) Berechnen eines Indikators des zerebralen Blutflusses ausgehend von dem Verhältnis.

2. Verfahren nach Anspruch 1, wobei das Finden der maximalen oder größten negativen Steigung das Finden der maximalen Steigung, sowohl für die IPG- als auch für die PPG-Signale umfasst, und das Finden eines Verhältnisses das Finden eines Verhältnisses der maximalen Steigungen umfasst.

3. Verfahren nach Anspruch 2, wobei die maximalen Steigungen Maxima in einem vorderen Abschnitt des Herzzyklus sind.

4. Verfahren nach Anspruch 1, wobei das Finden der maximalen oder größten negativen Steigung das Finden der größten negativen Steigung sowohl für die IPG- als auch für die PPG-Signale umfasst, und das Finden eines Verhältnisses das Finden eines Verhältnisses der größten negativen Steigungen umfasst.

5. Verfahren nach Anspruch 4, wobei die größten negativen Steigungen in einem hinteren Abschnitt des Herzzyklus am negativsten sind.

6. Verfahren nach einem der vorhergehenden Ansprüche, wobei die maximale oder größte negative Steigung mindestens eines der Signale auf eine Messung der Amplitude des Signals normiert ist.

7. Verfahren nach Anspruch 6, wobei die Messung der Amplitude die Amplitude von Spitze zu Spitze des Signals während des Herzzyklus ist.

8. Verfahren nach Anspruch 6, wobei die Messung der Amplitude ein durchschnittlicher Wert des Signals während des Herzzyklus ist.

9. Verfahren nach einem der vorhergehenden Ansprüche, wobei das PPG-Signal von einem PPG-Sensor auf der linken Seite des Kopfes stammt.

10. Verfahren nach einem der Ansprüche 1 bis 8, wobei das PPG-Signal von einem PPG-Sensor auf der rechten Seite des Kopfes stammt.

11. Verfahren nach einem der Ansprüche 1 bis 8, wobei das PPG-Signal ein Mittelwert der Signale von einem PPG-Sensor auf der linken Seite des Kopfes und von einem PPG-Sensor auf der rechten Seite des Kopfes ist.

12. Verfahren zum Schätzen eines zeitlich veränderlichen zerebralen Blutflusses, umfassend folgende Schritte:
a) Erzielen eines zeitlich veränderlichen IPG-, d.h. Impedanzplethysmographie, Signals vom Kopf;
b) Erzielen eines zeitlich veränderlichen PPG-, d.h. Photoplethysmographie, Signals vom Kopf;
c) Verwenden der IPG- und PPG-Signale zum Berechnen eines zeitlich veränderlichen Indikators für den zerebralen Blutfluss; und
d) Ausführen einer Datenverarbeitung an einem oder mehreren des IPG-Signals, des PPG-Signals und des Indikators des zerebralen Blutflusses, um Rauschen oder Artefakte, oder beides, zu reduzieren.

13. Verfahren nach Anspruch 12, wobei das Ausführen einer Datenverarbeitung das Aussondern von Daten des IPG-Signals, des PPG-Signals, oder von beiden, für die Herzzyklen, die einem oder mehreren Aussonderungskriterien entsprechen, umfasst.

14. Verfahren nach Anspruch 13, wobei die Kriterien das Aufweisen einer Dauer außerhalb eines erwarteten Bereichs umfassen.

15. Verfahren nach Anspruch 14, wobei der erwartete Bereich ein Maximum zwischen 1,3- und 2fache durchschnittliche Dauer von Herzzyklen aufweist.

16. Verfahren nach einem der Ansprüche 13 bis 15, wobei die Kriterien die Tatsache umfassen, dass eines oder zwei des IPG-Signals und des PPG-Signals eine Kreuzkorrelation unterhalb einer Schwelle zwischen diesem Herzzyklus und dem nächsten Herzzyklus aufweist bzw. aufweisen.

17. Verfahren nach einem der Ansprüche 13 bis 15, wobei die Kriterien die Tatsache umfassen, dass eines oder zwei des IPG-Signals und des PPG-Signals eine Kreuzkorrelation unterhalb einer Schwelle zwischen diesem Herzzyklus und dem vorhergehenden Herzzyklus aufweist bzw. aufweisen.

18. Verfahren nach Anspruch 16 oder 17, wobei die Schwelle zwischen +0,5 und +0,8 liegt.

19. Verfahren nach einem der Ansprüche 12 bis 18, wobei das Ausführen einer Datenverarbeitung das Reduzieren der Atmungsartefakte in dem IPG-Signal, dem PPG-Signal oder beiden umfasst.

20. Verfahren nach einem der Ansprüche 12 bis 19, wobei das Berechnen des Indikators des zerebralen Blutflusses die Verwendung des Verfahrens nach einem der Ansprüche 1 bis 11 umfasst.

21. Verfahren nach einem der Ansprüche 12 bis 20, wobei das Ausführen der Datenverarbeitung das Glätten des Indikators des zerebralen Blutflusses umfasst.

22. Verfahren nach Anspruch 21, wobei das Glätten das Finden eines Mittelwertes während eines Zeitintervalls umfasst.

23. Verfahren nach Anspruch 21 oder 22, wobei das Glätten die Verwendung einer Zeitskala umfasst, die je nach dem Verhalten des Indikators des zerebralen Blutflusses als Funktion der Zeit adaptiv angepasst wird.

## Revendications

1. Procédé d'estimation d'un flux sanguin cérébral par l'analyse de signaux IPG, c'est-à-dire de pléthysmographie à impédance, et PPG, c'est-à-dire de photopléthysmographie, de la tête, le procédé comprenant les étapes consistant à :
a) trouver une pente maximale ou une plus grande pente négative du signal IPG, à l'intérieur d'au moins une portion du cycle cardiaque ;
b) trouver une pente maximale ou une plus grande pente négative du signal PPG, à l'intérieur d'au moins une portion du cycle cardiaque ;
c) trouver un rapport entre la pente maximale ou la plus grande pente négative du signal IPG et la pente maximale ou la plus grande pente négative du signal PPG ; et
d) calculer un indicateur de flux sanguin cérébral à partir du rapport.

2. Procédé selon la revendication 1, dans lequel le fait de trouver la pente maximale ou la plus grande pente négative comprend le fait de trouver la pente maximale, à la fois pour les signaux IPG et PPG, et le fait de trouver un rapport comprend le fait de trouver un rapport des pentes maximales.

3. Procédé selon la revendication 2, dans lequel les pentes maximales sont maximales à l'intérieur d'une portion avant du cycle cardiaque.

4. Procédé selon la revendication 1, dans lequel le fait de trouver la pente maximale ou la plus grande pente négative comprend le fait de trouver la plus grande pente négative, à la fois pour les signaux IPG et PPG, et le fait de trouver un rapport comprend le fait de trouver un rapport des plus grandes pentes négatives.

5. Procédé selon la revendication 4, dans lequel les plus grandes pentes négatives sont les plus négatives à l'intérieur d'une portion arrière du cycle cardiaque.

6. Procédé selon l'une quelconque des revendications précédentes, dans lequel la pente maximale ou la plus grande pente négative d'au moins l'un des signaux est normalisée sur une mesure de l'amplitude dudit signal.

7. Procédé selon la revendication 6, dans lequel la mesure de l'amplitude est l'amplitude de crête à crête dudit signal durant le cycle cardiaque.

8. Procédé selon la revendication 6, dans lequel la mesure de l'amplitude est une valeur moyenne dudit signal durant le cycle cardiaque.

9. Procédé selon l'une quelconque des revendications précédentes, dans lequel le signal PPG provient d'un capteur PPG sur le côté gauche de la tête.

10. Procédé selon l'une quelconque des revendications 1 à 8, dans lequel le signal PPG provient d'un capteur PPG sur le côté droit de la tête.

11. Procédé selon l'une quelconque des revendications 1 à 8, dans lequel le signal PPG est une moyenne de signaux provenant d'un capteur PPG sur le côté gauche de la tête et d'un capteur PPG sur le côté droit de la tête.

12. Procédé d'estimation d'un flux sanguin cérébral variable dans le temps, comprenant les étapes consistant à :
a) obtenir un signal IPG, c'est-à-dire de pléthysmographie à impédance, variable dans le temps, de la tête ;
b) obtenir un signal PPG, c'est-à-dire de photopléthysmographie, variable dans le temps, de la tête ;
c) utiliser les signaux IPG et PPG pour calculer un indicateur variable dans le temps pour le flux sanguin cérébral ; et
d) effectuer un traitement de données sur un ou plusieurs du signal IPG, du signal PPG et de l'indicateur de flux sanguin cérébral, pour réduire du bruit ou des artefacts, ou les deux.

13. Procédé selon la revendication 12, dans lequel le fait d'effectuer un traitement de données comprend l'élimination de données du signal IPG, du signal PPG, ou des deux, pour des cycles cardiaques qui répondent à un ou plusieurs critères d'élimination.

14. Procédé selon la revendication 13, dans lequel les critères comprennent le fait d'avoir une durée en dehors d'une plage attendue.

15. Procédé selon la revendication 14, dans lequel la plage attendue présente un maximum entre 1,3 et 2 fois une durée moyenne des cycles cardiaques.

16. Procédé selon l'une quelconque des revendications 13 à 15, dans lequel les critères comprennent le fait qu'un ou deux du signal IPG et du signal PPG présente une corrélation croisée au-dessous d'un seuil, entre ce cycle cardiaque et le cycle cardiaque suivant.

17. Procédé selon l'une quelconque des revendications 13 à 15, dans lequel les critères comprennent le fait qu'un ou deux du signal IPG et du signal PPG présente une corrélation croisée au-dessous d'un seuil, entre ce cycle cardiaque et le cycle cardiaque précédent.

18. Procédé selon la revendication 16 ou 17, dans lequel le seuil se trouve entre +0,5 et +0,8.

19. Procédé selon l'une quelconque des revendications 12 à 18, dans lequel le fait d'effectuer un traitement de données comprend le fait de réduire des artefacts respiratoires dans le signal IPG, le signal PPG, ou les deux.

20. Procédé selon l'une quelconque des revendications 12 à 19, dans lequel le calcul de l'indicateur de flux sanguin cérébral comprend l'utilisation du procédé selon l'une quelconque des revendications 1 à 11.

21. Procédé selon l'une quelconque des revendications 12 à 20, dans lequel l'exécution du traitement de données comprend le lissage de l'indicateur de flux sanguin cérébral.

22. Procédé selon la revendication 21, dans lequel le lissage comprend le fait de trouver une moyenne sur un intervalle de temps.

23. Procédé selon la revendication 21 ou 22, dans lequel le lissage comprend l'utilisation d'une échelle chronologique qui est ajustée de façon adaptative, en fonction du comportement de l'indicateur de flux sanguin cérébral comme une fonction du temps.
